# EUROPÄISCHE PATENTSCHRIFT

(11) **EP 1 909 858 B1**
(45) Veröffentlichungstag und Bekanntmachung des Hinweises auf die Patenterteilung: **12.09.2018**
(21) Anmeldenummer: 06762799.2
(22) Anmeldetag: 25.07.2006
(51) Int. Cl.: A61L 15/60, A61L 15/22, A61L 26/00, A61P 17/02

(54) **HYDROGEL**
HYDROGEL
HYDROGEL

(30) Priorität: 30.07.2005 DE 102005035879
(43) Veröffentlichungstag der Anmeldung: 16.04.2008
(73) Patentinhaber: Paul Hartmann Aktiengesellschaft, 89522 Heidenheim (DE)
(72) Erfinder: GEIGER, Ralph, 34587 Felsberg (DE); GARRIGOS SISTARE, Gema, Tallahassee, FL 32303 (US); EFFING, Jochem, 65779 Kelkheim-Fischbach (DE)
(86) Internationale Anmeldenummer: PCT/EP2006/007308
(87) Internationale Veröffentlichungsnummer: WO 2007/014672

(56) Entgegenhaltungen:
- EP-A- 1 275 376
- WO-A-96/04025
- DE-A1- 10 012 026
- FR-A- 2 832 061
- JP-A- 6 145 060
- KUMAR S ET AL: "MODIFICATION OF IN SITU GELLING BEHAVIOR OF CARBOPOL SOLUTIONS BY HYDROXYPROPYL METHYLCELLULOSE" JOURNAL OF PHARMACEUTICAL SCIENCES, AMERICAN PHARMACEUTICAL ASSOCIATION. WASHINGTON, US, Bd. 84, Nr. 3, März 1995 (1995-03), Seiten 344-348, XP001109576 ISSN: 0022-3549

## Beschreibung

Die Erfindung betrifft einen Wundfüller umfassend ein Hydrogel sowie dessen Herstellung und die Verwendung in der modernen Wundbehandlung.

Hydrogele sind bereits seit einiger Zeit dem Anwender als Wundbehandlungsmittel bekannt. Diese Produkte zeichnen sich durch einen hohen Wassergehalt aus und sind besonders geeignet, in der feuchten Wundbehandlung eingesetzt zu werden. Angeboten werden Hydrogele in Form von gefriergetrockneten Pads, durchsichtigen Kompressen oder als amorphe Gele in Tuben und Spritzen.

In der Patentliteratur sind Hydrogele zur Wundbehandlung beispielsweise durch die europäischen Patentschrift EP 382 128 B1 bekannt. Hierin wird ein vernetztes wundsekretabsorbierendes Hydrogel beschrieben, das durch Vernetzung eines natürlichen Gelierungsmittels ausgewählt aus der Gruppe Collagen, Gelatine, Pektin und/oder Alginate mit einem Copolymer aus einer oder mehreren Vinylcarbonsäuren und wenigstens einem ihrer Salze hergestellt wird. Neben den mittels eines Vernetzungsmittels vernetzten Polymeren enthält das Hydrogel weiterhin einen mehrwertigen Alkohol sowie Wasser oder eine physiologische Kochsalzlösung.

Ein weiteres Hydrogel zur Anwendung als Wundverband ist in der EP 848 621 B1 beschrieben, das ein "bacteriostatic agent" und Fasern umfasst. Die Fasern sollen in das Gel eingearbeitet sein und Kationen zur Vernetzung des Hydrogels bereitstellen. Das Hydrogel soll zudem eine Viskosität von 20.000 bis 1.000.000 cPs aufweisen.

Weiterhin wird mit der EP 987 019 A1 ein Hydrogel zur Behandlung von Wunden beschrieben, das seinerseits eine Zusammensetzung aufweist, die in halbfester Form vorliegt. Die Zusammensetzung umfasst neben Wasser und einem Antibiotikum auch Partikel, die in der Lage sind, mindestens 30 Gew.-% ihres Eigengewichtes an Wasser aufzunehmen und mindestens 70 Gew.-% ihres Eigengewichtes an Wasser abzugeben. Die halbfeste Zusammensetzung soll neben einem Polyol mindestens zwei Geliermittel umfassen.

Mit der DE 100 12 026 A1 werden ein Gel sowie die Verwendung eines Gels zum Lösen von Verkrustungen von Wunden beschrieben. Das beschriebene wässrige Gel soll ausgeprägte mikrobiozide Wirkung aufweisen und Polyhexamethylguanidin sowie Glycerin und Hydroxyethylcellulose enthalten. Des Weiteren kann das Gel als wässrige Lösung eine Kochsalz- oder Ringer-Lösung aufweisen.

Ein Hydrogel zur Anwendung als Wundverband ist darüber hinaus in der europäischen Patentschrift EP 576 523 B2 beschrieben. Dieses Hydrogel besteht aus einem wasserunlöslichen, in Wasser quellbaren vernetzten Cellulosederivat, Wasser und einer Polyolkomponente. Das mit dieser Patentschrift beschriebene Gel betrifft in erster Linie die Entfernung von nekrotischem Gewebe, da es die Notwendigkeit des Einsatzes eines chemischen Debridierungsmittels oder chirurgischen Herausschneidens verringert.

Diese durch den Stand der Technik bekannten Hydrogele weisen insgesamt oder teilweise Eigenschaften auf, die vom Anwender eher als nachteilig empfunden werden oder sie enthalten Inhaltsstoffe, die vom Anwender in manchen Behandlungsfällen als eher kritisch eingeschätzt oder in einem fortschreitenden Wundheilungsverlauf als störend angesehen werden. Daher ist es eine Aufgabe der vorliegenden Erfindung, ein Hydrogel bereitzustellen, das in der modernen Wundbehandlung einzusetzen ist, das in der Anwendung einer Wunde ein wundheilungsförderndes Klima bereitstellt und das die Nachteile der bekannten Hydrogele vermeidet. Dabei soll das Hydrogel sowohl bei trockenen Wunden als auch bei Wundsekret absondernden Wunden angewendet werden können und bei der Behandlung dieser verschiedenen Wunden eine schonende Debridierung gewährleisten. Weiterhin ist es eine Aufgabe der vorliegenden Erfindung ein Hydrogel bereitzustellen, das schmerzlindernd wirkt und in der Anwendung ein Gleichgewicht zwischen Wunde und Wundbehandlungsmittel bereitstellt. Eine weitere Aufgabe besteht darin, ein Hydrogel als Wundfüller zur Verfügung zu stellen, das im applikationsfähigen Zustand eine gute Modellierbarkeit gewährleistet und bei Aufnahme von Wundexsudat einen guten Zusammenhalt aufweist und keinen nennenswerten Abbau der Viskosität zeigt. Darüber hinaus ist es eine Aufgabe, ein Verfahren bereitzustellen, mit dem ein wundheilungsförderndes Hydrogel hergestellt werden kann.

Gelöst werden diese Aufgaben durch einen Wundfüller gemäß Anspruch 1 umfassend ein Hydrogel. Weiterhin wird zur Lösung der Aufgabe ein Verfahren zur Herstellung eines Wundfüllers gemäß Anspruch 7 vorgeschlagen.

Ein Vorteil des in dem Wundfüller enthaltenen Hydrogels besteht darin, dass durch die Kombination von gelbildenden Polysacchariden zusammen mit einem Acrylsäurederivates ein Hydrogel bereit gestellt werden kann, das je nach Wundentyp sowohl saugende Eigenschaft - bei stark nässenden Wunden - als auch wasserabgebende Eigenschaft - bei trockenen Wunden - aufweist, und das sehr gut modellierbar ist. Überraschender Weise hat sich gezeigt, das ein solches Hydrogel auch mittels elektromagnetischer Strahlung oder Elektronen- oder Positronenstrahlung sterilisierbar ist. Werden nämlich gelbildende Polysaccharide zur Bildung des Hydrogels alleine verwendet, so wird nach der Sterilisation mittels Strahlen oder Teilchen ein Gel erhalten, dass nur eine unzureichende Viskosität und eine unzureichende Wasseraufnahmekapazität aufweist. Wird auf der anderen Seite ein Gel alleine aus Acrylsäurederivaten aufgebaut, wird ein Gel erhalten, das zwar durch Strahlen oder Teilchen sterilisierbar ist, jedoch eine unzureichende Modellierfähigkeit besitzt und zudem eine schlechte Wasserabgabefähigkeit aufweist. Zudem kann einer Wunde mit einem erfindungsgemäßen Hydrogel durch das Elektrolyt-Gemisch ein überaus wundheilungsförderndes Klima bereitgestellt werden, da durch die mindestens zwei Elektrolyte ein dem Wundserum ähnliches Elektrolytgemisch bereit gestellt werden kann. Mit diesem Hydrogel kann darüber hinaus ein Wundbehandlungsmittel bereitgestellt werden, das ein in einer Wunde vorliegende nekrotisches Gewebe aufbricht und eine schonende Debridierung gewährleistet. Diese Debridierung ist die Vorraussetzung für einen natürlichen Zellaufbau, der ausgehend von den Wundrändern einen kontinuierlichen Heilungsverlauf kennzeichnet.

In einer bevorzugten Ausführungsform weist das erfindungsgemäße Hydrogel kein Konservierungsmittel auf. Darüber hinaus soll ein erfindungsgemäßes Hydrogel insbesondere keine antimikrobiell, fungizid, antibakteriell oder in einer anderen Weise Pilze, Mikroben, Bakterien, Viren im Wachstum hemmende oder abtötende Wirkstoffe umfassen.

Gemäß einer besonders bevorzugten Ausführungsform umfasst ein Hydrogel vorzugsweise mindestens ein gelbildendes Polysaccharid ausgewählt aus der Gruppe der Cellulose-Derivate oder deren Salze, Alginate oder deren Derivate, Chitin oder dessen Derivate oder dessen Salze oder Stärke. Hierbei ist der Ursprung der gelbildenden Polysaccharide unbeachtlich, das heißt, dass diese gelbildenden Polysaccharide pflanzlichen oder tierischen Ursprungs sein können oder auf synthetischem Weg beispielsweise durch mikrobiologische Verfahren hergestellt sein können. Es ist auch möglich Polysaccharide zu verwenden, die pflanzlichen oder tierischen Ursprungs und durch chemische Synthese modifiziert sind.

Zu der Gruppe der Cellulose-Derivate zählen im Zusammenhang mit der vorliegenden Erfindung insbesondere Celluloseether und Celluloseester sowie deren Salze. Als Celluloseether kommen hierbei insbesondere Hydroxyalkylcellulosen insbesondere Hydroxy-C₁₋₆-alkylcellulose wie zum Beispiel Hydroxymethylcellulose, Hydroxyethylcellulose, Hydroxypropylcellulose oder Hydroxybutylcellulose und ganz besonders bevorzugt Hydroxymethylcellulose oder Hydroxyethylcellulose zum Einsatz. Als Celluloseester kommen hierbei insbesondere Carboxyalkylcellulose insbesondere Carboxy-Ci-e-alkylcellulose wie zum Beispiel Carboxymethylcellulose, Carboxyethylcellulose, Carboxypropylcellulose oder Carboxybutylcellulose und ganz besonders bevorzugt Carboxymethylcellulose oder Carboxyethylcellulose zum Einsatz.

Gemäß einer zweiten bevorzugten Ausführungsform umfasst das Hydrogel mindestens zwei verschiedene gelbildende Polysaccharide. Dabei hat es sich als besonders vorteilhaft erwiesen, wenn die mindestens zwei Polysaccharide ausgewählt werden aus der Gruppe der Cellulose-Derivate oder deren Salze insbesondere Celluloseether und Celluloseester, Alginate oder deren Derivate, Chitin oder dessen Derivate oder dessen Salze oder Stärke. Besonders bevorzugt umfasst ein erfindungsgemäßes Hydrogel hierbei mindestens zwei Polysaccharide aus der Gruppe der Celluloseether und Celluloseester. Ganz besonders bevorzugt ist ein Hydrogel, das als gelbildende Polysaccharide Hydroxyalkylcellulose und Carboxyalkylcellulose enthält.

Weiterhin kann ein Hydrogel als gelbildendes Polysaccharid insbesondere mindestens ein wasserlösliches Cellulose-Derivat umfassen. Ganz besonders bevorzugt umfasst dieses Hydrogel mindestens zwei verschiedene gelbildende, wasserlösliche Polysaccharide. Diese Polysaccharide zeichnen sich dadurch aus, dass sie innerhalb des Gels keine gequollenen Partikel bilden wodurch wiederum ein sehr homogenes Hydrogel resultiert. Zudem zeigt ein wasserlösliches Polysaccharid umfassendes Hydrogel bei der Auftragung auf einer Wunde eine besonders gute Streichfähigkeit auf, bildet zudem eine besonders glatte Oberfläche aus und ist besonders gut modellierbar. Ganz besonders bevorzugt werden hierbei wasserlösliche Cellulose-Derivate, die nicht quervernetzt sind.

Es kann jedoch auch vorgesehen sein, dass das erste gelbildende Polysaccharid ausgewählt wird aus der Gruppe der Cellulose-Derivate insbesondere Hydroxyalkylcellulose oder Carboxyalkylcellulose und das zweite gelbildende Polysaccharid ausgewählt wird aus der Gruppe der Alginate, insbesondere Natrium-, Kalium- oder Kalziumalginat, und/oder Chitin oder dessen Derivate oder Salze.

Bei der Verwendung von insbesondere zwei gelbildenden Polysacchariden ist weiterhin besonders bevorzugt ein erstes nichtionisches Polysaccharid und mindestens ein zweites ionisches Polysaccharid einzusetzen. Es kann aber auch vorgesehen sein, das zwei nichtionische Polysaccharide oder zwei ionische Polysaccharide Verwendung finden. Hierbei können insbesondere als nichtionisches Polysaccharid wasserlösliche Celluloseether und ganz besonders bevorzugt wasserlösliche Hydroxyalkylcellulosen eingesetzt werden. Als ionische Polysaccharide können hierbei insbesondere wasserlösliche Celluloseester und ganz besonders bevorzugt wasserlösliche Carboxyalkylcellulosen eingesetzt werden. Es kann aber auch vorgesehen sein, als ionisches Polysaccharid ein Alginat oder ein Gemisch aus verschiedenen Alginaten wie beispielsweise Natrium-, oder Kalziumalginat einzusetzen.

Das in einem erfindungsgemäßen Hydrogel vorhandene Acrylsäure-Derivat dient in erster Linie als Strukturbildner oder Viskositätsverbesserer. Hierzu sind besonders Polyacrylsäure und deren Salze und insbesondere quervernetzte Polyacrylate geeignet. Diese Polyacrylsäure-Derivate weisen zudem den Vorteil auf, dass sie einen erheblichen Anteil ihres Eigengewichtes an Wasser aufnehmen können. Durch die Kombination dieser Acrylsäure-Derivate mit mindestens einem gelbildenden Polysaccharid lässt sich somit gezielt ein Hydrogel herstellen, dessen Wasseraufnahme- und Wasserabgabekapazität gesteuert werden kann.

Des Weiteren ist vorgesehen, dass das Hydrogel eine dynamische Viskosität von 5.000 bis 60.000 mPa s, insbesondere 5.000 bis 50.000 mPa s und ganz besonders 10.000 bis 40.000 mPa s aufweist (gemessen Bohlin-Rheometer Typ CSR - 10, Kegelspindel 4° / ∅ 40 mm, Spaltabstand 100 µm, Oszillometrische Messung, T= 22-27 °C). Ein solches Hydrogel lässt sich besonders gut und gleichmäßig beispielsweise mit einem Spachtel über und in einer Wunde verteilen, weist auch bei der Aufnahme von Wundexsudat einen guten Zusammenhalt auf und fließt nicht aus einer zu behandelnden Wunde.

In einer besonders bevorzugten Ausführungsform umfasst das Hydrogel mindestens ein gelbildendes Polysaccharid und ein Acrylsäure-Derivat, wobei das Gewichtsverhältnis des oder der Polysaccharide zu dem Acrylsäure-Derivat in dem Hydrogel dem Verhältnis von 20:1 bis 1:1, insbesondere 15:1 bis 1:1 und ganz besonders 10:1 bis 1:1 entspricht.

Weiterhin ist vorgesehen, dass das Hydrogel bezogen auf das Gesamtgewicht des Hydrogels mindestens 50 Gew.-% Wasser, 0,5-10 Gew.-% gelbildendes Polysaccharid, 0,05-6,0 Gew.-% Acrylsäure-Derivat und 0,001-4,0 Gew.-% Elektolyt-Gemisch umfasst. Insbesondere umfasst das Hydrogel mindestens 50 Gew.-% Wasser, 1-6 Gew.-% gelbildendes Polysaccharid, 0,5-4,0 Gew.-% Acrylsäure-Derivat und 0,001-2,0 Gew.-% Elektolyt-Gemisch.

Wird dem Hydrogel neben einem ersten gelbilden Polysaccharid ein zweites gelbildendes Polysaccharid zugesetzt, so umfasst dieses Hydrogel bezogen auf das Gesamtgewicht des Hydrogels mindestens 50 Gew.-% Wasser, 0,5-5 Gew.-% erstes gelbildendes Polysaccharid, 0,5-5 Gew.-% zweites gelbildendes Polysaccharid, 0,05-6,0 Gew.-% Acrylsäure-Derivat und 0,001-4,0 Gew.-% Elektolyt-Gemisch. Insbesondere umfasst das Hydrogel mindestens 50 Gew.-% Wasser, 0,5-4 Gew.-% erstes gelbildendes Polysaccharid, 0,5-4 Gew.-% zweites gelbildendes Polysaccharid, 0,5-4,0 Gew.-% Acrylsäure-Derivat und 0,001-2,0 Gew.-% Elektolyt-Gemisch.

Werden mindestens zwei verschiedene gelbildende Polysaccharide eingesetzt, so kann insbesondere vorgesehen sein, dass das Gewichtsverhältnis des ersten zu dem zweiten Polysaccharid zueinander dem Verhältnis von 1:6 bis 6:1 und insbesondere 1:4 bis 4:1 entspricht. Wird dabei, wie erfindungsgemäß vorgesehenals erstes gelbildendes Polysaccharid Hydroxyethylcellulose und als zweites gelbildendes Polysaccharid Carboxymethylcellulose eingesetzt, so kann über das Verhältnis dieser beiden Komponenten weiterhin die Wasseraufnahme- bzw. Wasserabgabekapazität des Gels eingestellt werden. Wird beispielsweise der Anteil an Carboxymethylcellulose gleich eins und der Anteil an Hydroxyethylcellulose größer als eins gesetzt, so wird im Vergleich zu einem Hydrogel, das gleiche Anteile der beiden Cellulosederivate enthält, ein Hydrogel erhalten, das eine höhere Wasserabgabe zeigt. Wird dagegen der Anteil an Hydroxyalkylcellulose gleich eins und der Anteil an Carboxymethylcellulose größer als eins gesetzt, so wird im Vergleich zu einem Hydrogel, das gleiche Anteile der beiden Cellulosederivate enthält, ein Hydrogel erhalten, das eine höhere Wasseraufnahme zeigt. Die Testungen zur Wasseraufnahmebeziehungsweise Wasserabgabekapazität werden dabei analog zu St. Thomas und P. Hay, Ostomy/ Wound Management1995, Vol. 41, Nr.3 S.54-59 durchgeführt.

In einer weiteren Ausführungsform ist vorgesehen, dass ein erfindungsgemäßes Hydrogel ein sterilisiertes Hydrogel ist, insbesondere ist dabei vorgesehen, dass das Hydrogel durch elektromagnetische Strahlung oder Elektronen- oder Positronenstrahlung sterilisiert ist. Das Hydrogel kann jedoch auch mittels Dampfsterilisation sterilisiert sein.

Für den Fall das ein erfindungsgemäßes Hydrogel mittels elektromagnetischer Strahlung oder Elektronen- oder Positronenstrahlung sterilisiert ist, ist weiterhin bevorzugt vorgesehen, dass dieses Hydrogel eine dynamische Viskosität von 5.000 bis 60.000 mPa s, insbesondere 5.000 bis 40.000 mPa s und ganz besonders 10.000 bis 40.000 mPa s (Bohlin-Rheometer Typ CSR - 10, Kegelspindel 4° / ∅ 40 mm, Spaltabstand 100 µm, Oszillometrische Messung) auf. Auch ein solches Hydrogel lässt sich besonders gut und gleichmäßig beispielsweise mit einem Spachtel über und in einer Wunde verteilen. Dieses Hydrogel weist auch bei der Aufnahme von Wundexsudat einen guten Zusammenhalt auf und fließt nicht aus einer zu behandelnden Wunde.

Geeignete Elektrolyte in Zusammenhang mit der vorliegenden Erfindung sind Verbindungen, die insbesondere beim Auflösen in Wasser, zur Dissoziation in Ionen befähigt sind und die aus mono-, di- und/ oder trivalenten Ionen aufgebaut sind. Diese Elektrolyte können beispielsweise als anorganische oder organische Salze vorliegen und sind in jedem Fall verschieden von den ebenfalls von dem vorliegenden Hydrogel umfassten Polymeren, die eventuell ionischen Charakter aufweisen. Besonders geeignet sind in diesem Zusammenhang Chloride, lodide, Sulfate, Hydrogensulfate, Karbonate, Hydrogenkarbonate, Phosphate, Dihydrogenphosphate oder Hydrogenphosphate der Alkali- und Erdalkalimetalle. Insbesondere finden als Elektrolyt-Gemisch in einem erfindungsgemäßen Hydrogel Natrium-, Kalium- und Kalziumchlorid Verwendung. Dieses Elektrolytgemisch simuliert in besonders guter Weise das Elektrolyt-Gemisch in einem von einer Wunde abgegebenen Wundserum. Damit stellt ein dieses Elektrolytgemisch enthaltendes Hydrogel einer Wunde ein besonders wundheilungsförderndes Klima zur Verfügung.

In einer besonders bevorzugten Ausführungsform weist das Hydrogel eine Leitfähigkeit von mindestens 4000 µS cm⁻¹ insbesondere mindestens 6000 µS cm⁻¹ und ganz besonders bevorzugt 6000- 20000 µS cm⁻¹. Die Leitfähigkeit ist insbesondere durch die Menge an Elektrolyt-Gemisch einzustellen. Dieses Einstellen ist vorteilhaft, da die Leitfähigkeit eines Hydrogels von den Komponenten des Gels und ihren Konzentrationen abhängt. Beispielsweise ist die Leitfähigkeit abhängig von der Art und der Konzentration des eingesetzten gelbildenden Polymers oder von der Art und der Konzentration des eingesetzten Polyols. Diese Komponenten setzten in unterschiedlichem Maß die Leitfähigkeit eines Hydrogels im Vergleich zu einer reinen Salzlösung herunter. Somit muss die Menge an Elekrolyt-Gemisch individuell an die übrigen Inhaltsstoffe des Gels angepasst werden, um eine bestimmte Leitfähigkeit einzustellen.

Es kann auch vorgesehen sein, dass das Hydrogel eine solche Menge an Elektrolyt-Gemisch aufweist, dass das Hydrogel seinerseits eine freie lonenkonzentation aufweist, die der freien Ionenkonzentration einer physiologischen Elektrolytlösung bzw. einer isotonischen Elektrolytlösung entspricht. Alternativ hierzu kann das Hydrogel auch ein isotonisches Hydrogel sein.

Insbesondere kann auch vorgesehen sein, dass das Hydrogel Ringerlösung umfasst. Hierbei soll unter einer Ringerlösung eine isotonische Salzlösung verstanden sein, die Natriumchlorid, Kaliumchlorid und Kalziumchlorid enthält.

In einer weiteren alternativen Ausgestaltung der Erfindung weist das Hydrogel zusätzlich ein Polyol auf. Dieses Polyol ist hervorragend als Feuchtigkeitsspender geeignet und stellt somit für die der Wunde umgebenden Haut eine pflegende Komponente dar. Insbesondere sind hierzu Polyole geeignet, die ausgewählt werden aus der Gruppe Glycerin, Glykol, Propylenglycol, Polyethylenglykol, Polypropylenglycol, Polyethylenpropylenglykol, Sorbitol, Sorbit oder deren Mischungen. Erfindungsgemäß wird als Polyol in dem vorliegenden Hydrogel Glycerin eingesetzt.

Wird dem Hydrogel ein Polyol zugesetzt so umfasst dieses Hydrogel bezogen auf das Gesamtgewicht des Hydrogels mindestens 50 Gew.-% Wasser, 5-30 Gew.-% Polyol, 0,5-10 Gew.-% gelbildendes Polysaccharid, 0,05-6,0 Gew.-% Acrylsäure-Derivat und 0,001-4,0 Gew.-% Elektolyt-Gemisch. Insbesondere weist das Hydrogel mindestens 50 Gew.-% Wasser, 10-30 Gew.-% Polyol, 1-6 Gew.-% gelbildendes Polysaccharid, 0,5-4,0 Gew.-% Acrylsäure-Derivat und 0,001-2,0 Gew.-% Elektolyt-Gemisch auf.

Wird dem Hydrogel in einer Weiterbildung des Erfindungsgedankens neben einem Polyol und einem ersten gelbildenden Polysaccharid zusätzlich ein zweites gelbildendes Polysaccharid zugesetzt so umfasst dieses Hydrogel bezogen auf das Gesamtgewicht des Hydrogels mindestens 50 Gew.-% Wasser, 5-30 Gew.-% Polyol, 0,5-5 Gew.-% erstes gelbildendes Polysaccharid und 0,5-5 Gew.-% zweites gelbildendes Polysaccharid, 0,05-6,0 Gew.-% Acrylsäure-Derivat und 0,001-4,0 Gew.-% Elektolyt-Gemisch. Insbesondere weist das Hydrogel dann mindestens 50 Gew.-% Wasser, 10-30 Gew.-% Polyol, 0,5-4 Gew.-% erstes gelbildendes Polysaccharid und 0,5-4 Gew.-% zweites gelbildendes Polysaccharid, 0,5-4,0 Gew.-% Acrylsäure-Derivat und 0,001-2,0 Gew.-% Elektolyt-Gemisch auf.

Neben dem Hydrogel selbst ist auch die Herstellung eines Hydrogels, insbesondere eines oben beschriebenen Hydrogels, Gegenstand der vorliegenden Erfindung. Das erfindungsgemäße Verfahren umfasst dabei folgende Verfahrensschritte:
a) Bereitstellen einer wässrigen Lösung umfassend ein Acrylsäure-Derivat,
b) Zugeben einer Suspension umfassend mindestens ein pulverförmiges Polysaccharid zu der unter a) bereitgestellten Lösung und
c) Bestrahlen der unter b) hergestellten Zusammensetzung mit elektromagnetischer Strahlung oder Elektronen- oder Positronenstrahlung zur Einstellung der Viskosität des Hydrogels.

Gemäß einer Weiterbildung des Verfahrens wird die unter a) bereitgestellte wässrige Lösung umfassend ein Acrylsäure-Derivat auf einen pH-Wert zwischen 5,5 und 7,0 eingestellt. Gemäß einem besonders bevorzugten Verfahren umfasst die unter b) genannte Suspension weiterhin ein Polyol, insbesondere ein flüssiges Polyol. Weiterhin bevorzugt wird diese Suspension hergestellt, indem das pulverförmige Polysaccharid in ein bei Raumtemperatur flüssiges Polyol eingerührt wird. Insbesondere ist dabei vorgesehen, die unter b) zuzugegebene Suspension herzustellen, indem das mindestens eine Polysacharid in ein bei Raumtemperatur flüssiges Polyol eingerührt wird und die unter a) genannte wässrige Lösung vor dem Zugeben der unter b) hergestellten Suspension einen pH-Wert zwischen 5,5 und 7,0 einzustellen. Des Weiteren ist vorgesehen, dass insbesondere die unter a) angeführte wässrige Lösung ein Elektrolyt-Gemisch umfasst, das seinerseits mindestens zwei Elektrolyte umfasst. Weiterhin bevorzugt kann die Bestrahlung insbesondere mittels β-Strahlung durchgeführt werden. Dabei sind insbesondere Dosen von 20-35 kGy vorteilhaft.

Damit ist weiterhin eine Zusammensetzung Gegenstand der vorliegenden Erfindung, die zur Herstellung eines mittels elektromagnetischer Strahlung oder Elektronen- oder Positronenstrahlung sterilisierten Hydrogels geeignet ist und mindestens ein gelbildendes Polysaccharid, ein Acrylsäurederivat und ein mindestens zwei verschiedene Elekrolyte umfassendes Elektrolyt-Gemisch umfasst und eine dynamische Viskosität von höchstens 40.000 mPa s (gemessen Bohlin-Rheometer Typ CSR - 10, Kegelspindel 4° / ∅ 40 mm, Spaltabstand 100 µm, Oszillometrische Messung, T= 22-27 °C) besitzt.
Ein erfindungsgemäßes Hydrogel ist insbesondere zur Behandlung von vergleichsweise tiefen Wunden geeignet und lässt sich hervorragend als Wundfüller einsetzen. So lassen sich beispielsweise tiefe dermale Ulzera, die sehr häufig stark nässend sind mit diesem Hydrogel behandeln. Hierbei wird das Heraussickern von Flüssigkeit aus der Wunde verhindert oder wenigstens verringert und gleichzeitig durch die Bereitstellung eines Elektrolyt-Gemischs ein Wundbehandlungsmittel bereitgestellt, das die Wundheilung fördert. Darüber hinaus können mit dem vorliegenden Gel aber auch trockene Wunden wie beispielsweise trockener Ulcus curis behandelt werden. Hierbei zeigt das vorliegende Hydrogel seine Fähigkeit der Wunde Flüssigkeit zuzuführen und die Entfernung von unerwünschten Substanzen, Belägen und Nekrosen durch eine schonende Debridierung zu gewährleisten. Unterstützt wird der Wundheilungsprozess durch einen semi-occlusiven Abschluss mittels eines sekundären Wundverbandes wie beispielsweise eines Filmverbandes wodurch unerwünschten Kontaminationen verhindert werden. Andere Kategorien von Wunden, für die das Gel angewendet werden kann, umfassen ohne darauf beschränkt zu sein Dekubitus Stufe I, II, III (Druckgeschwür), Ulcus cruris (Unterschenkelgeschwür, offenes Bein), diabetisches Fußsyndrom, Hautgeschwüre, Blutgeschwüre, Verbrennungen ersten und zweiten Grades, Schürfwunden und chronische Wunden. Damit betrifft die vorliegende Erfindung auch die Verwendung eines Hydrogels, das mindestens ein gelbildendes Polysaccharid, ein Acrylsäure-Derivat und ein Elektrolyt-Gemisch umfasst, wobei das Elektrolyt-Gemisch mindestens zwei verschiedene Elektrolyte aufweist, zur Herstellung eines Mittels zur Wundheilung insbesondere zur Behandlung von Dekubitus Stufe I, II, III (Druckgeschwür) oder Ulcus cruris (Unterschenkelgeschwür, offenes Bein) oder diabetisches Fußsyndrom oder Hautgeschwüre oder Blutgeschwüre oder Verbrennungen ersten und zweiten Grades oder Schürfwunden oder chronische Wunden.

In einer besonderen Ausgestaltung der Erfindung ist weiterhin vorgesehen, dass das erfindungsgemäße Hydrogel in einer Verpackung angeordnet ist. Insbesondere ist dabei vorgesehen, dass das Hydrogel in der Verpackung steril verpackt ist. Hierbei können Verpackungen wie beispielsweise Behälter mit Schraubverschluss, verschließbare Tuben oder Behälter zum Einmalverbrauch wie Tuben mit Sicherheitsverschluss Anwendung finden. Es kann jedoch auch vorgesehen sein, dass das Hydrogel in einer Spritze, die als Erstverpackung verwendet wird, angeordnet ist. Insbesondere ist hierbei vorgesehen, dass das Hydrogel in der Spritze steril vorliegt. In einer besonders bevorzugten Ausführung liegt dieses in einer sterilen Erstverpackung vorliegende Hydrogel als Set fertig zur Anwendung in der weiteren Verpackung zusammen mit medizinischem Träger- oder Verbandmaterial und gegebenenfalls weiteren medizinischen Hilfsmitteln vor. Hierbei kann auch vorgesehen sein, dass sowohl das Hydrogel in einer Erstverpackung als auch das medizinische Träger- oder Verbandmaterial in einer sterilen Erstverpackung zusammen in der Set-Verpackung vorliegen.

### Beispiele:

### 1) Beispiele 1 bis 4

### a) Zusammensetzung Beispiel 1 bis 4

| | Hydrogel 1 * | Hydrogel 2 * | Hydrogel 3 | Hydrogel 4 |
|---|---|---|---|---|
| Hydroxyethylcellulose ⁽¹⁾ | 300 g | 300 g | 300 g | 300 g |
| Carboxymethylcellulose ⁽²⁾ | | 100 g | 100 g | 100 g |
| Polyacrylat ⁽³⁾ | 70 g | 70 g | 70 g | 70 g |
| Glycerin ⁽⁴⁾ | 2000 g | 2000 g | 2000 g | 2000 g |
| Natriumchlorid (p.a.) | | | | 84 g |
| Kalziumchlorid Dihydrat (p.a.) | | | | 3,1 g |
| Kaliumchlorid (p.a.) | | | | 2,9 g |
| 1N-Natronlauge (p.a.) | 750 g | 750 g | 750 g | 750 g |
| Wasser, gereinigt Ph.Eur. | | 6780 g | | 6690 g |
| Ringer-Lösung ⁽⁵⁾ | 6880 g | | 6780 g | |
| pH-Wert ^{(A)} | 6,0 | 6,0 | 6,0 | 6,0 |
| Viskosität ^{(B)} / Pa s: | | | | |
| a) nicht steril | 120 | 195 | 190 | 200 |
| b) steril ^{(D)} | | | 27 | 22 |
| Leitfähigkeit ^{(C)} / µS cm⁻¹: | | | | |
| a) nicht steril | 7460 | 1456 | 8020 | 10300 |
| b) steril ^{(D)} | | | | 10733 |

| | | | | |
|---|---|---|---|---|
| * nicht erfindungsgemäße Hydrogele ⁽¹⁾ Hydroxyethylcellulose (HEC) Natrosol HX Pharm (Fa. Hercules, Rijswijk - Niederlande) ⁽²⁾ Carboxymethylcellulose (CMC) Blanose 7H4 (Fa. Hercules, Rijswijk - Niederlande) ⁽³⁾ Polyacrylat Carbopol 980 NF (Fa. Noveon, Calvert City - USA) ⁽⁴⁾ Glycerin (wasserfrei) (Fa. DOW Deutschland Inc.) ⁽⁵⁾ Ringer-Lösung: 1000 ml Lösung enthalten: 8,60 g Natriumchlorid (NaCl) 0,30 g Kaliumchlorid (KCl) 0,33 g Kalziumchlorid-Dihydat (CaCl₂ * 2H₂O) Rest Wasser, gereinigt Ph.Eur. (H₂O) ^{(A)} Der pH-Wert wird bestimmt mit einem pH-Meter Typ CG 841 (Fa. Schott - Deutschland) bestückt mit einer Glaselektrode SenTix 81 (Fa. Wissenschaftlich-Technische Werkstätten GmbH, Weilheim - Deutschland). Die Proben sind vor den Messungen auf 25°C temperiert und werden bei 25 °C Raumtemperatur vermessen. ^{(B)} Die Viskosität wird gemessen mit Bohlin-Rheometer Typ CSR - 10 (Fa. Bohlin Instruments, Mühlacker - Deutschland), Kegelspindel 4° / ∅ 40 mm, Spaltabstand 100 µm, Oszillometrische Messung. Die Proben sind vor den Messungen auf 25 °C temperiert und werden bei 25 °C vermessen. ^{(C)} Die Leitfähigkeit wird gemessen mit einer Standard-Leitfähigkeitsmesszelle Tetracon 325 (Fa. Wissenschaftlich-Technische Werkstätten GmbH, Weilheim - Deutschland). Die Proben sind vor den Messungen auf 25 °C temperiert und werden bei 22-25 °C vermessen. ^{(D)} Die sterilen Hydrogele sind mittels β-Sterilisation sterilisiert. Die Dosisverteilung liegt je nach Bestrahlungsanordnung der Gelmuster zwischen 25 bis 36 kGy. Um eine geringe Dosisverteilung zu erreichen wurden die Proben in 55 ml Plastikdosen (Durchmesser 55 mm, Material PP) mit 30 gr. Gel befüllt. Als steril wurde eine Minimumbestrahlung von 25 kGy angesetzt. | | | | |

### b) Herstellung der Hydrogele 1 bis 4

Die Hydogele 1 bis 4 sind alle gemäß den aufgeführten Arbeitsschritten bei Raumtemperatur hergestellt worden. Zur Herstellung der Hydrogele wird in einem ersten Schritt eine Suspension aus pulverförmigem Polysaccharid (HEC oder ein Gemisch aus HEC und CMC) und Glycerin hergestellt, indem das Polysaccharid langsam in Glycerin gegeben und gleichmäßig verrührt wird. In einem zweiten Schritt wird eine Lösung des Polyacrylats hergestellt. Hierzu wird die oben angegebene Menge pulverförmiges Polyacrylat in die Ringer-Lösung, in Wasser oder in die wässrige Elektrolytlösung gegeben und zwei Stunden gerührt. Unter Zugabe der Natronlauge wird ein pH-Wert von pH = 6 eingestellt und weitere zwei Stunden gerührt. In die auf pH = 6 eingestellte Lösung wird im Folgenden sehr langsam unter gleichmäßigen Rühren die Suspension aus HEC/Glycerin oder HCE/CMC/Glycerin gegeben. Nach der Zugabe wird mindestens zwei weitere Stunden bei Raumtemperatur gerührt. Bei der Herstellung kann Luft in das Gel eingearbeitet werden. Diese Einschlüsse können durch Rühren im Vakuum wieder entfernt werden. Die resultierenden Hydrogele werden in Tuben gefüllt und mittels β-Sterilisation sterilisiert. Die Dosisverteilung liegt je nach Bestrahlungsanordnung der Gelmuster zwischen 25 bis 36 kGy. Durch die Bestrahlung wird die Viskosität auf ungefähr 1/10 der Viskosität des unsterilen Hydrogels eingestellt.

### c) Beschreibung der Hydrogele

Die resultierenden Hydrogele 1 bis 4 (die Hydrogele 1 und 2 sind keine anspruchsgemäßen Hydrogele) sind insgesamt transparente, amorphe Hydrogele, die eine gute bis sehr gute Modellierbarkeit aufweisen. Dabei zeigen die Hydrogele 3 und 4 im unsterilen Zustand eine hohe Viskosität und im sterilen Zustand eine niedrigere Viskosität (ca. 1/10 der unsterilen Hydrogele). Zudem kann insbesondere mit dem Hydrogel 1, 3 und 4 ein Hydrogel zur Wundbehandlung bereitgestellt werden, das einer Wunde eine dem Wundserum ähnliche Elektrolyt-Zusammensetzung enthält. Die Veränderungen der Leitfähigkeit zwischen unsteril und sterilem Gel liegen innerhalb der Messtoleranzen.

### 2) Beispiele 5 bis 8

### a) Zusammensetzung Beispiel 3 und 5 bis 8

Die Hydrogele 3, 5 bis 8 enthalten alle die im Beispiel Hydrogel 3 angegebene Zusammensetzung mit dem Unterschied, das als Ringer-Lösung eine zur Ringer-Lösung ähnliche Lösung verwendet wird.

| | Konzentration der Ringerähnlichen Lösungen ^{(E)} | Leitfähigkeit (nicht steril) |
|---|---|---|
| Ringer-Lösung | | 16150 µS cm ⁻¹ |
| Hydrogel 5 | x = 0,5 | 4120 µS cm⁻¹ |
| Hydrogel 3 | x = 1,0 = Ringer-Lösung | 8020 µS cm⁻¹ |
| Hydrogel 6 | x = 1,5 | 11140 µS cm⁻¹ |
| Hydrogel 7 | x = 2,0 | 12620 µS cm⁻¹ |
| Hydrogel 8 | x = 2,5 | 16370 µS cm⁻¹ |

| | | |
|---|---|---|
| ^{(E)} 1000 ml Lösung einer jeweils zur Ringer-ähnlichen Lösung enthalten: x mal 8,60 g Natriumchlorid (NaCl) x mal 0,30 g Kaliumchlorid (KCl) x mal 0,33 g Kalziumchlorid-Dihydat (CaCl₂ * 2H₂O) Rest Wasser, gereinigt Ph.Eur. (H₂O) | | |

### b) Herstellung der Hydrogele 5 bis 8

Die Hydrogele 5 bis 8 werden analog zu den Hydrogelen 1 bis 4 hergestellt.

### c) Beschreibung der Hydrogele

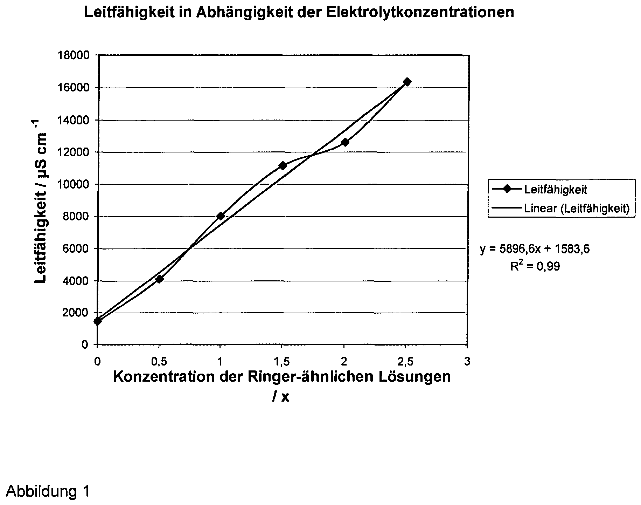

Die Hydrogele 3, 5 bis 8 enthalten alle die im Beispiel Hydrogel 3 angegebene Zusammensetzung mit dem Unterschied, das als Ringer-Lösung eine zur Ringer-Lösung analoge Lösung verwendet wird. Diese Lösungen unterscheiden sich untereinander lediglich durch den Gehalt an Elektrolyt-Gemisch. Damit weist z.B. das Hydrogel 7 im Vergleich zum Hydrogel 3 eine doppelte Menge an Elektrolyt-Gemisch auf. Die Leitfähigkeiten der verschiedenen Gele unterscheiden sich beträchtlich. Insbesondere weist das Hydrogel 3, das Ringer-Lösung enthält lediglich eine halb so hohe Leitfähigkeit auf, wie die Ringer-Lösung selbst. Eine Leitfähigkeit analog zu einer Ringerlösung wird erst mit dem Hydrogel 8 erreicht. Dieses Hydrogel 8 weist im Vergleich zum Hydrogel 3 eine 2,5-fache Menge an Elektrolyt-Gemisch auf. Das Hydrogel 8 kann als isotonisches Hydrogel bezeichnet werden, da in dieses Hydrogel dieselbe Leitfähigkeit aufweist, wie eine isotonische Ringer-Lösung. Die Messung der Leitfähigkeit erfolgte im unsterilen Zustand. Der Anstieg der Leitfähigkeit ist annähernd linear, wie dies der Abbildung 1 entnommen werden kann. Somit kann auch gezeigt werden, dass mit einer einmal festgelegten Zusammensetzung die Leitfähigkeit durch Variation der Elektrolytmenge gesteuert werden kann.

### Zusammenfassung

Die vorliegende Erfindung betrifft einen Wundfüller umfassend ein Hydrogel sowie dessen Herstellung und die Verwendung in der modernen Wundbehandlung. Das Hydrogel umfasst mindestens 50 Gew.-% Wasser.

## Patentansprüche

1. Wundfüller umfassend ein Hydrogel mit einem Wassergehalt von mindestens 50 Gew.-% bezogen auf das Gesamtgewicht des Hydrogels, wobei das Hydrogel
a) mindestens zwei verschiedene gelbildende Polysaccharide umfassend Hydroxyethylcellulose und Carboxymethylcellulose,
b) mindestens ein Acrylsäure-Derivat, wobei das Acrylsäure-Derivat ein vernetztes Polyacrylat ist,
c) ein Elektrolyt-Gemisch, wobei das Elektrolyt-Gemisch Natrium-, Kalium- und Kalziumchlorid enthält,
und
d) Glycerin
umfasst,
und wobei das Gewichtsverhältnis der Polysaccharide a) zu dem Acrylsäure-Derivat b) dem Verhältnis von 20:1 bis 1:1 entspricht,
und wobei das Hydrogel einen pH-Wert zwischen 5 und 7 aufweist.

2. Wundfüller nach Anspruch 1, **dadurch gekennzeichnet, dass** das Hydrogel bezogen auf das Gesamtgewicht des Hydrogels 0,5 - 5 Gew.-% des ersten gelbildenden Polysaccharids, 0,5 - 5 Gew.-% des zweiten gelbildenden Polysaccharids, 0,05 - 6,0 Gew.-% Acrylsäure-Derivat und 0,001 - 4,0 Gew.-% des Elektrolyt-Gemisches umfasst.

3. Wundfüller nach Anspruch 1 oder 2, **dadurch gekennzeichnet, dass** das Hydrogel eine Leitfähigkeit von mindestens 4000 µS cm-1 aufweist, wobei die Leitfähigkeit durch das Elektrolyt-Gemisch einstellbar ist.

4. Wundfüller nach mindestens einem der vorgenannten Ansprüche, **dadurch gekennzeichnet, dass** das Hydrogel Ringerlösung umfasst, wobei die Ringerlösung eine isotonische Salzlösung ist, die Natriumchlorid, Kaliumchlorid und Kalziumchlorid enthält.

5. Wundfüller nach mindestens einem der vorgenannten Ansprüche, **dadurch gekennzeichnet, dass** das Hydrogel durch elektromagnetische Strahlung oder Elektronen- oder Positronenstrahlung sterilisiert ist.

6. Wundfüller nach mindestens einem der vorgenannten Ansprüche, **dadurch gekennzeichnet, dass** das Hydrogel eine dynamische Viskosität von 5.000 bis 60.000 mPa s aufweist.

7. Verfahren zur Herstellung eines Wundfüllers umfassend ein Hydrogel, mit den folgenden Verfahrensschritten
a) Herstellen einer Suspension aus einem pulverförmigen Polysaccharid und Glycerin, wobei es sich bei dem pulverförmigen Polysaccharid um ein Gemisch aus Hydroxyethylcellulose und Carboxymethylcellulose handelt, und wobei das Polysaccharid langsam in Glycerin gegeben und gleichmäßig verrührt wird,
b) Bereitstellen einer wässrigen Lösung eines Polyacrylates, wobei hierfür pulverförmiges Polyacrylat in eine Ringer-Lösung gegeben und zwei Stunden gerührt wird, nachfolgend Einstellen des pH-Wertes auf pH 6 unter Zugabe von Natronlauge und Rühren für weitere zwei Stunden,
c) Zugeben der Suspension zu der unter b) bereitgestellten wässrigen Lösung, wobei das Zugeben sehr langsam und unter gleichmäßigem Rühren erfolgt, danach mindestens zwei weitere Stunden Rühren bei Raumtemperatur und
d) Bestrahlen der unter c) hergestellten Zusammensetzung mittels β-Sterilisation zur Einstellung der Viskosität des Hydrogels auf ungefähr 1/10 der Viskosität des unsterilen Hydrogels, wobei die Dosisverteilung zwischen 25 bis 36 kGy liegt.

8. Verwendung eines Hydrogels nach mindestens einem der vorgenannten Ansprüche, zur Herstellung eines Mittels zur Wundheilung insbesondere zur Behandlung von Dekubitus Stufe I, II, III (Druckgeschwür) oder Ulcus cruris (Unterschenkelgeschwür, offenes Bein) oder diabetischem Fußsyndrom oder Hautgeschwüren oder Blutgeschwüren oder Verbrennungen ersten und zweiten Grades oder Schürfwunden oder chronischen Wunden.

## Claims

1. Wound filler comprising a hydrogel having a water content of at least 50% by weight, based on the total weight of the hydrogel, wherein the hydrogel comprises
a) at least two different gel-forming polysaccharides comprising hydroxyethylcellulose and carboxymethylcellulose,
b) at least one acrylic acid derivative, wherein the acrylic acid derivative is a crosslinked polyacrylate,
c) an electrolyte mixture, wherein the electrolyte mixture comprises sodium chloride, potassium chloride and calcium chloride,
and
d) glycerol,
and wherein the ratio by weight of the polysaccharides a) to the acrylic acid derivative b) corresponds to the ratio of from 20:1 to 1:1,
and wherein the hydrogel has a pH of between 5 and 7.

2. Wound filler according to Claim 1, **characterized in that** the hydrogel, based on the total weight of the hydrogel, comprises from 0.5 to 5% by weight of the first gel-forming polysaccharide, from 0.5 to 5% by weight of the second gel-forming polysaccharide, from 0.05 to 6.0% by weight acrylic acid derivative and from 0.001 to 4.0% by weight of the electrolyte mixture.

3. Wound filler according to Claim 1 or 2, **characterized in that** the hydrogel has a conductivity of at least 4000 µS cm-1, wherein the conductivity is adjustable by means of the electrolyte mixture.

4. Wound filler according to at least one of the above-mentioned claims, **characterized in that** the hydrogel comprises Ringer's solution, wherein the Ringer's solution is an isotonic solution which comprises sodium chloride, potassium chloride and calcium chloride.

5. Wound filler according to at least one of the above-mentioned claims, **characterized in that** the hydrogel is sterilized by electromagnetic radiation or electron or positron radiation.

6. Wound filler according to at least one of the above-mentioned claims, **characterized in that** the hydrogel has a dynamic viscosity of from 5000 to 60,000 mPa s.

7. Method for producing a wound filler comprising a hydrogel, having the following method steps
a) preparing a suspension of a polysaccharide in powder form and glycerol, wherein the polysaccharide in powder form is a mixture of hydroxyethylcellulose and carboxymethylcellulose, and wherein the polysaccharide is slowly added to glycerol and stirred constantly,
b) providing an aqueous solution of a polyacrylate, wherein for this purpose polyacrylate in powder form is added to a Ringer's solution and stirred for two hours, then adjusting the pH to pH 6 with the addition of sodium hydroxide solution and stirring for a further two hours,
c) adding the suspension to the aqueous solution provided under b), wherein the addition takes place very slowly and with constant stirring, and then stirring for at least a further two hours at room temperature, and
d) irradiating the composition prepared under c) by means of β sterilization in order to adjust the viscosity of the hydrogel to approximately 1/10 of the viscosity of the non-sterile hydrogel, wherein the dose distribution is between 25 and 36 kGy.

8. Use of a hydrogel according to at least one of the preceding claims in the production of an agent for wound healing, in particular for the treatment of stage I, II, III bedsores (pressure ulcers) or Ulcus cruris (leg ulcer, ulcerated leg) or diabetic foot syndrome or skin ulcers or boils or first- and second-degree burns or abrasions or chronic wounds.

## Revendications

1. Agent de remplissage de plaies comprenant un hydrogel ayant une teneur en eau d'au moins 50 % en poids par rapport au poids total de l'hydrogel, dans lequel l'hydrogel comprend
a) au moins deux différents polysaccharides gélifiants, comprenant de l'hydroxyéthylcellulose et de la carboxyméthylcellulose,
b) au moins un dérivé d'acide acrylique, le dérivé d'acide acrylique étant un polyacrylate réticulé,
c) un mélange d'électrolytes, le mélange d'électrolytes contenant du chlorure de sodium, du chlorure de potassium et du chlorure de calcium,
et
d) du glycérol,
et dans lequel le rapport pondéral des polysaccharides a) au dérivé d'acide acrylique b) correspond à un rapport valant de 20:1 à 1:1,
et dans lequel l'hydrogel présente un pH compris entre 5 et 7.

2. Agent de remplissage de plaies selon la revendication 1, **caractérisé en ce que** l'hydrogel comprend par rapport au poids total de l'hydrogel 0,5 - 5 % en poids du premier polysaccharide gélifiant, 0,5 - 5 % en poids du deuxième polysaccharide gélifiant, 0,05 - 6,0 % en poids de dérivé d'acide acrylique et 0,001 - 4,0 % en poids du mélange d'électrolytes.

3. Agent de remplissage de plaies selon la revendication 1 ou 2, **caractérisé en ce que** l'hydrogel présente une conductivité d'au moins 4 000 µS cm⁻¹, la conductivité étant ajustable au moyen du mélange d'électrolytes.

4. Agent de remplissage de plaies selon au moins l'une quelconque des revendications précédentes, **caractérisé en ce que** l'hydrogel comprend de la solution de Ringer, la solution de Ringer étant une solution de sels isotonique qui contient du chlorure de sodium, du chlorure de potassium et du chlorure de calcium.

5. Agent de remplissage de plaies selon au moins l'une quelconque des revendications précédentes, **caractérisé en ce que** l'hydrogel est stérilisé par irradiation électromagnétique ou irradiation par faisceau d'électrons ou de positrons.

6. Agent de remplissage de plaies selon au moins l'une quelconque des revendications précédentes, **caractérisé en ce que** l'hydrogel présente une viscosité dynamique de 5 000 à 60 000 mPa.s.

7. Procédé pour la préparation d'un agent de remplissage de plaies comprenant un hydrogel, comportant les étapes de procédé suivantes
a) préparation d'une suspension à partir d'un polysaccharide pulvérulent et de glycérol, le polysaccharide pulvérulent consistant en un mélange d'hydroxyéthylcellulose et de carboxyméthylcellulose et en introduisant lentement le polysaccharide dans le glycérol et tout en délayant,
b) disposition d'une solution aqueuse d'un polyacrylate, en introduisant à cet effet du polyacrylate pulvérulent dans une solution de Ringer et en agitant pendant deux heures, puis en ajustant le pH à pH 6 par addition d'une solution d'hydroxyde de sodium et en agitant pendant encore deux heures,
c) addition de la suspension à la solution aqueuse fournie en b), l'addition s'effectuant très lentement et sous agitation simultanée, puis encore au moins deux heures d'agitation à la température ambiante et
d) irradiation de la composition préparée en c) par stérilisation aux rayons β pour l'ajustement de la viscosité de l'hydrogel à environ 1/10 de la viscosité de l'hydrogel non stérile, la distribution de doses étant comprise entre 25 et 36 kGy.

8. Utilisation d'un hydrogel selon au moins l'une quelconque des revendications précédentes, pour la préparation d'une composition destinée à la guérison de plaies, en particulier au traitement d'ulcère de décubitus de grade I, II, III (escarre de décubitus) ou d'ulcère de la jambe (jambe ulcérée) ou du syndrome du pied diabétique ou d'ulcères de la peau ou de furoncles ou de brûlures aux premier et second degrés ou d'excoriations ou de plaies chroniques.
